# EUROPEAN PATENT SPECIFICATION

(11) **EP 3 332 694 B1**
(45) Date of publication and mention of the grant of the patent: **01.04.2020**
(21) Application number: 16202985.4
(22) Date of filing: 08.12.2016
(51) Int. Cl.: A61B 5/00, A61B 5/11, A61B 5/0482

(54) **HANDHELD TOOL FOR LEVELING UNCOORDINATED MOTION**
HANDWERKZEUG ZUM NIVELLIEREN UNKOORDINIERTER BEWEGUNG
OUTIL À MAIN DE NIVELLEMENT DE MOUVEMENT NON COORDONNÉ

(43) Date of publication of application: 13.06.2018
(73) Proprietor: Verily Life Sciences LLC, Mountain View, CA 94043 (US)
(72) Inventor: Pathak, Anupam, Mountain View, CA 94043 (US); Allen, Michael, Mountain View, CA 94043 (US)
(74) Representative: Mewburn Ellis LLP

(56) References cited:
- KR-B1- 101 659 554
- US-A1- 2013 297 022
- US-A1- 2014 052 275
- US-A1- 2016 242 679

## Description

### TECHNICAL FIELD

This disclosure relates generally to tools for leveling or stabilizing muscle movements.

### BACKGROUND INFORMATION

Motor impairment is a partial or total loss of function of a body part, usually a limb. This is often caused by muscle weakness, poor stamina, or a lack of motor control. It is often a symptom of neurological disorders such as Parkinson's Disease, ALS, stroke, Multiple Sclerosis, or Cerebral Palsy. There are few, if any effective, technologies available to assist with motor impairment and limitations in movement. As a result, many individuals are unable to conduct simple tasks such as feeding themselves, forcing them to rely on a caregiver.

US 2016/242679 A1 discloses a technique for measuring and collecting human tremor data which includes measuring motions of a handheld tool manipulated by a user while performing a task with the handheld tool. The motions are measured using an inertial measurement unit ("IMU") disposed within a handle of the handheld tool. Motion data of the motions is recorded to a motion log stored within a memory unit of the handheld tool. The motion data contains information for determining a severity of tremors that occurred while the user performed the task with the handheld tool. The motion log is communicated to a remote server for analysis.

KR 101 659 554 B1 discloses a spoon having a balancing function. The spoon having a balancing function, according to an embodiment of the present invention, comprises: a head part which is formed in a concave shape to store food; a body part which is connected to the rear end of the head part to be used as a grip; a measurement unit which senses the rotation movement of the body part; a rotating unit which provides driving force for rotating the head part around a roll axis and a pitch axis; a control part which receives the measurement value of the measurement unit and outputs a drive signal corresponding to the measurement value to the rotating unit; and a battery which supplies power to the measurement unit and the rotating unit.

The present invention provides a handheld tool according to claim 1. The present invention also provides a method executed by a handheld tool according to claim 12. Further aspects of the present invention are set out in the remaining claims.

### BRIEF DESCRIPTION OF THE DRAWINGS

Non-limiting and non-exhaustive embodiments of the invention are described with reference to the following figures, wherein like reference numerals refer to like parts throughout the various views unless otherwise specified. The drawings are not necessarily to scale, emphasis instead being placed upon illustrating the principles being described.
FIG. 1A is a perspective view illustration of a handheld tool that provides auto-leveling to a user-assistive device, in accordance with an embodiment of the disclosure.
FIG. 1B is a cutaway perspective view illustration of a handheld tool that provides auto-leveling to a user-assistive device, in accordance with an embodiment of the disclosure.
FIG. 1C is a plan view illustration of a handheld tool that provides auto-leveling to a user-assistive device, in accordance with an embodiment of the disclosure.
FIG. ID is a side view illustration of a handheld tool that provides auto-leveling to a user-assistive device, in accordance with an embodiment of the disclosure.
FIG. 2 is a functional block diagram illustrating components of system circuitry of a handheld tool that provides auto-leveling to a user-assistive device, in accordance with an embodiment of the disclosure.
FIG. 3 is a functional block diagram illustrating components of a motion control system for providing auto-leveling to a user-assistive device of a handheld tool, in accordance with an embodiment of the disclosure.
FIG. 4 is a perspective view illustration of a handheld tool with a user-assistive device fashioned to hold a cup for drinking, in accordance with an embodiment of the disclosure.

### DETAILED DESCRIPTION

Embodiments of an apparatus, system, and method of operation for providing auto-leveling of a user-assistive device of a handheld tool are described herein. In the following description numerous specific details are set forth to provide a thorough understanding of the embodiments. One skilled in the relevant art will recognize, however, that the techniques described herein can be practiced without one or more of the specific details, or with other methods, components, materials, etc. In other instances, well-known structures, materials, or operations are not shown or described in detail to avoid obscuring certain aspects.

Reference throughout this specification to "one embodiment" or "an embodiment" means that a particular feature, structure, or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

Technologies to help with human tremors have been developed, but they may be unsuitable for a variety of conditions where the human tremor is too extreme in magnitude, or the motor impairment results in tipping/spilling due to lack of muscle control. Stabilized platforms using inertial measurement units ("IMU") have been developed for cameras (e.g., brushless gimbal controllers) both in military applications and for hobbyists. Stabilized flight controllers similarly stabilize a moving platform in three-dimensional space. However, these solutions are not viable for integration into a small lightweight handheld tool to help people with muscle strength or muscle control limitations perform everyday tasks such as eating, drinking, or otherwise. Furthermore, certain occupations (e.g., surgical field) can benefit from tool leveling and/or stabilization particularly in high stress environments like an operating room or even a mobile army surgical hospital.

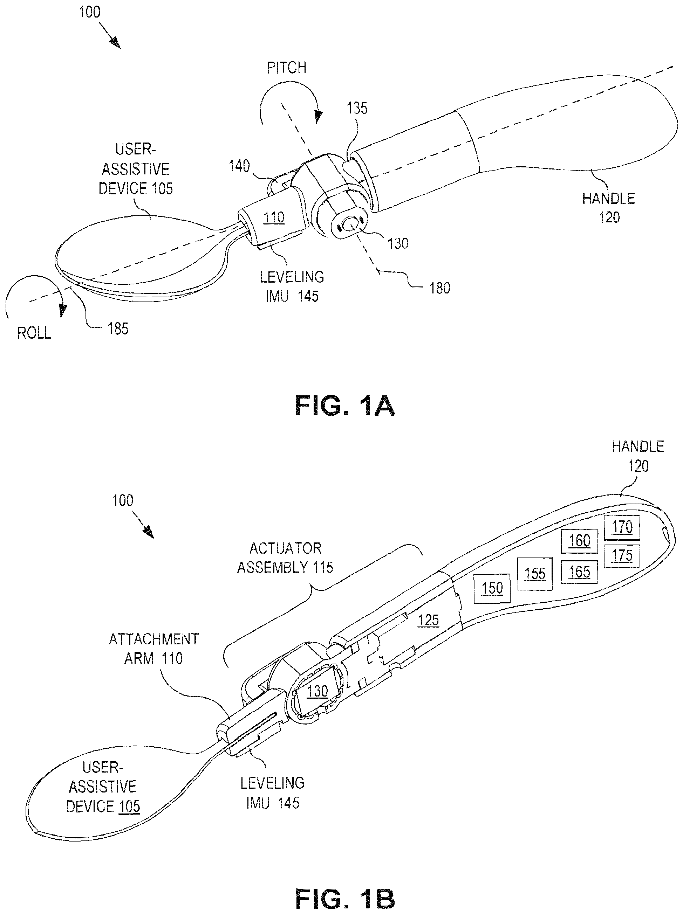

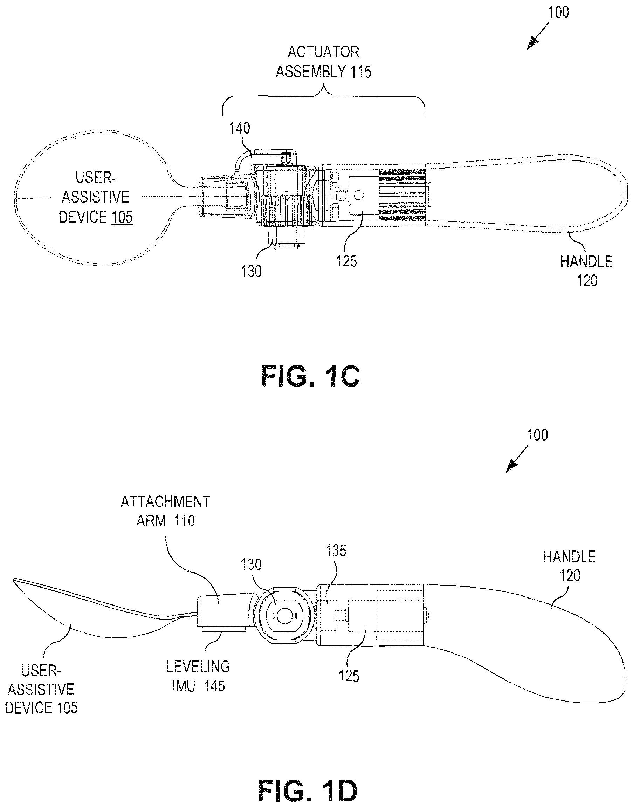

FIGs. 1A-D illustrate a handheld tool 100 that is capable of auto-leveling, and in some embodiments stabilizing, a user-assistive device 105 connected to an end of handheld tool 100, in accordance with an embodiment of the disclosure. FIG. 1A is a perspective view illustration of handheld tool 100 while FIG. 1B is a cutaway perspective view illustration, FIG. 1C is a plan view illustration, and FIG. 1D is a side view illustration all of the same embodiment of handheld tool 100. The illustrated embodiment of handheld tool 100 includes a user-assistive device 105, an attachment arm 110, an actuator assembly 115, a handle 120, and a system circuitry. The illustrated embodiment of actuator assembly 115 includes actuator 125, actuator 130, linkage 135, and linkage 140. System circuitry includes a leveling IMU 145, a motion control system 150, a power supply 155, position sensors (not illustrated in FIGs. 1A-1D), a system controller 160, system memory 165, and a communication interface 170. In one embodiment, handheld tool 100 may also include a tremor IMU 175.

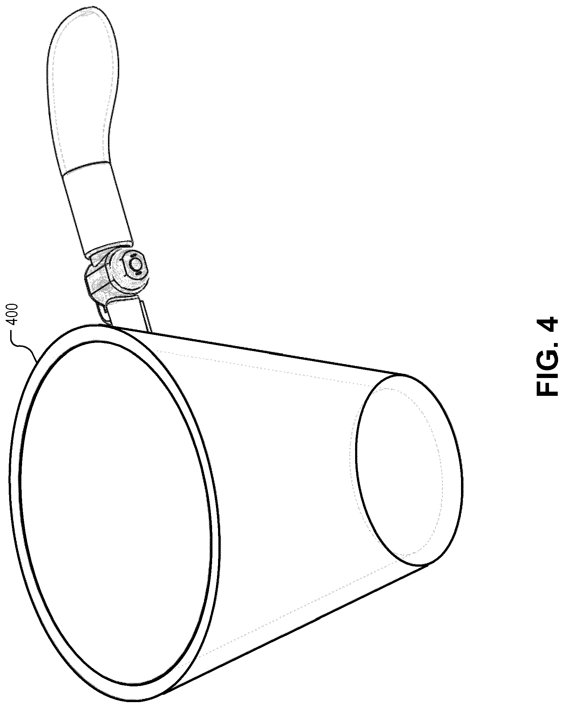

Handheld tool 100 is an auto-leveling (and in some embodiments tremor stabilizing) platform that can be adapted to hold a variety of different user-assistive devices 105. Handheld tool 100 provides active leveling using electronic actuators and a feedback control system. FIGs. 1A-D illustrates user-assistive device 105 as a spoon; however, user-assistive device 105 may be implemented as a variety of different eating utensils, drinking utensils (e.g., see cup-holder device 400 in FIG. 4), a makeup applicator, a pointing device, various occupational tools (e.g., surgical tools), or otherwise.

The illustrated embodiment of handheld tool 100 includes leveling IMU 145 disposed on attachment arm 145, which is rigidly connected to user-assistive device 105 to measure motions and orientation of user-assistive device 105. Leveling IMU 145 outputs feedback data indicative of the measured motions and orientation to motion control system 150. Leveling IMU 145 may be implemented with a gyroscope and accelerometer, or even additionally include a magnetometer. In one embodiment, leveling IMU 145 is a solid-state device.

In one embodiment, motion control system 150 polls leveling IMU 145 for linear accelerations, angular velocity, and orientation relative to a frame of reference (e.g., gravity vector) of user-assistive device 105 at a given instant. Motion control system 150 then executes an algorithm to estimate the orientation of user-assistive device 105 in three-dimensional ("3D") space relative to the frame of reference. This estimation or estimated vector of gravity relative to the body-frame of the leveling IMU (and user-assistive device 105) is continually updated in real-time and used to generate command signals for driving and controlling actuator assembly 115 in real-time. In one embodiment, the command signals include a roll command and a pitch command.

Actuator assembly 115 is connected to user-assistive device 105 to manipulate user-assistive device 105 in at least two orthogonal dimensions. In the illustrated embodiment, the two orthogonal dimensions include rotation about a pitch axis 180 and rotation about a roll axis 185. The pitch axis 180 is orthogonal to roll axis 185, which runs longitudinally through handle 120. In other embodiments, the two motion dimensions need not be orthogonal. Furthermore, in yet other embodiments, additional degrees of freedom may be added to actuator assembly 115 such as linear motions or even a yaw rotation.

Actuator assembly 115 is present in handheld tool 100 to move attachment arm 110 and by extension user-assistive device 105 relative to handle 120 for auto-leveling, and in some embodiments, tremor stabilization. If user-assistive device 105 is pitched or rolled relative to the fixed reference frame (e.g., gravity vector), the motion control system 150 will command actuator assembly 115 to move user-assistive device 105 in opposite directions to compensate and retain a level orientation or even provide an offsetting orientation to counteract a tremor. The overall effect is user-assistive device 105 remains fixed in orientation (or even stabilized), regardless of how the handle is oriented within physical limits of actuator assembly 115.

The illustrated embodiment of actuator assembly 115 includes actuator 125 which provides output rotational motion about roll axis 185. This roll motion is coupled to actuator 130 via a linkage 135 such that actuator 125 physically rotates actuator 130 about roll axis 185. The illustrated embodiment of actuator 130 provides output rotational motion about pitch axis 180. The pitch and roll motions are coupled to attachment arm, and by extension user-assistive device 105, via linkage 140 such that actuator 130 pitches user-assistive device 105 while actuator 125 rolls user-assistive device 105. These orthogonal rotational motions are independently controlled.

In one embodiment, handheld tool 100 further includes two position sensors that provide feedback positional information to motion control system 150 that is indicative of the rotational positions of the outputs of actuators 125 and 130 relative to handle 120. In other words, the positional sensors indicate the positions of linkages 135 and 140 relative to handle 120. In one embodiment, each positional sensor is a hall-effect sensor that monitors the positions of its respective linkage 135 or 140. Other positional sensors may be implemented including potentiometers, encoders, etc.

Conventional stabilizing devices attempt to provide stabilization using a weighted pendulum. However, a heavy mass is required to force the platform to rest in a level state. Disadvantages to such implementations include a required bulk and mass and the potential of swinging or oscillating of the pendulum at its natural frequency. The set - point (stabilized position) of the user assistive device is also limited by the mechanical assembly and cannot be easily adjusted. Furthermore, data about the user cannot be collected through these purely mechanical means. In contrast, the feedback control system used in handheld tool 100 can achieve much greater performance in a significantly smaller form-factor. Heavy weights are not required, and motion control system 150 can be specially tuned to react to various unintended motion (e.g., tremor stabilization). In fact, motion control system 150 can be programmed to respond to both uncoordinated movements (low frequency) for auto-leveling and unintentional movements (high frequency) for stabilization of human tremors.

Additionally, system controller 160 can be programmed to monitor and collect data about the severity of the user's condition (e.g., ability to maintain a level orientation, amount of feedback control assistance needed, amount of unintentional tremor motions, etc.) and store this data into a log within system memory 165 for eventual output via communication interface 170. The log can be analyzed and provided to a healthcare provider to diagnose and treat the user/patient's condition. The active control provided by motion control system 150 can further be programmed to automatically adjust in small increments overtime as part of a therapy plan. The therapy plan can be monitored using the log and tailored on a per patient basis by referring to the log. For example, the amount of active leveling/stabilization may be incrementally reduced at a prescribed rate as a sort of therapy or training and the results periodically monitored with reference to the log.

In one embodiment, attachment arm 110 is implemented as a permanent, fixed connection to a single user-assistive device 105. In other embodiments, attachment arm 110 may facilitate a non-permanent attachment to remove or replace user-assistive devices 105. Using a non-permanent attachment enables the user to insert or attach different types of user-assistive devices 105 to handheld tool 100. For example, user-assistive device 105 may be implemented as a variety of different eating or drinking utensils (e.g., spoon, knife, fork, cup-holder), personal hygiene tools (e.g., toothbrush, floss pick), grooming tools (e.g., makeup applicator, comb), occupational tools (e.g., surgical tools), pointing devices (e.g., laser pointer or stick pointer), or otherwise. The auto-leveling (and optional tremor stabilization) functionality can help users who have uncoordinated (and/or unintentional) muscle movements to have improved quality of life by providing greater independence and self-control over routine tasks. Furthermore, handheld tool 100 may have occupational uses that aid those that do not suffer from uncoordinated/unintentional muscle movements.

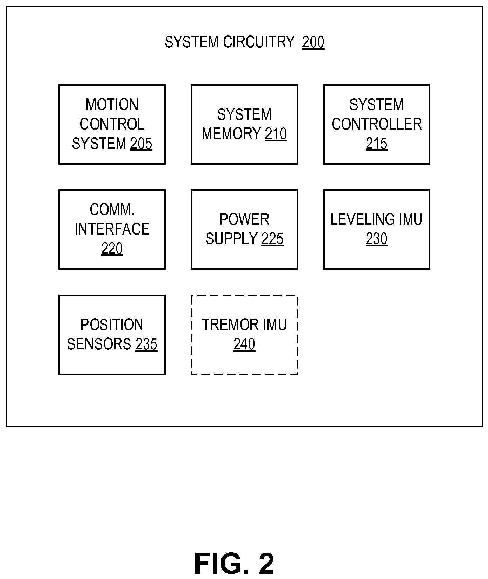

FIG. 2 is a functional block diagram illustrating functional components of system circuitry 200, in accordance with an embodiment of the disclosure. System circuitry 200 illustrates example functional control components for the operation of handheld tool 100. The illustrated embodiment of system circuitry 200 includes a motion control system 205, system memory 210, a system controller 215, a communication interface 220, a power supply 225, a leveling IMU 230, position sensors 235, and a tremor IMU 240.

As discussed above, motion control system 205 receives (e.g., polls) feedback data from leveling IMU 230 to determine the orientation and motion of user-assistive device 105. This feedback data is analyzed using a control algorithm to generate commands for manipulating actuator assembly 115. In one embodiment, motion control system 205 is implemented as digital signal processing ("DSP") circuit. In another embodiment, motion control system 205 is software/firmware logic executed on system controller 215 and stored in system memory 210. In one embodiment, system controller 215 is implemented as a microprocessor and system memory 210 is nonvolatile memory (e.g., flash memory). Other types of memory and controllers may be used.

In one embodiment, communication interface 220 is communicatively coupled to system controller 215 to output data (e.g., usage log) stored in system memory 210. Communication interface 220 may be implemented as a wired or wireless interface, such as a universal serial port ("USB"), a wireless Bluetooth interface, a WiFi interface, a cellular interface, or otherwise.

As mentioned above, leveling IMU 230 is disposed to monitor the orientation and motion of user-assistive device 105. In the illustrated embodiment of FIGs. 1A-D, leveling IMU 145 is disposed on attachment arm 145. In an embodiment where user-assistive device 105 is permanently fixed to handheld tool 100, leveling IMU 230 may also be rigidly mounted to user-assistive device 105 itself or attachment arm 110 may be considered an extension piece of user-assistive device 105. Leveling IMU 230 may be implemented as a solid-state sensor including one or more of an accelerometer, a gyroscope, or a magnetometer.

Position sensors 235 are relative sensors that measure the relative positions of the outputs of actuator assembly 115 relative to handle 120. In one embodiment, position sensors 235 are hall-effect sensors that monitor the position of the outputs of actuators 125 and 130 by measuring the positions of linkages 135 and 140. The relative position information output by position sensors 235 may be recorded to a log within system memory 210 for determining how much auto-leveling a user needs and thereby diagnosing the severity and progress of a given user.

In one embodiment, handheld tool 100 may further include tremor IMU 240 rigidly mounted to handle 120 to measure the motion/orientation of handle 100. The tremor feedback information acquired by tremor IMU 240 may also be recorded to a log file within system memory 210 to facilitate diagnosis and treatment of a user's condition. In some embodiments, feedback data from tremor IMU 240 may also be used for feedback stabilization, though feedback data from leveling IMU 230 may be sufficient and even preferable for both auto-leveling and stabilization of user-assistive device 100.

In the illustrated embodiment, the functional components of system circuitry 200 are powered by power supply 225. In one embodiment, power supply 225 is a rechargeable battery (e.g., lithium ion battery) disposed within handle 120 of handheld tool 100. Many of the other functional components of system circuitry 200 may also be disposed within handle 120 to provide a compact, user friendly form factor. For example, in various embodiments, some or all of motion control system 205, system memory 210, system controller 215, communication interface 220, power supply 225, and tremor IMU 240 are disposed within handle 120. As illustrated in FIGs. 1A-D, actuator 125 and linkage 135 are at least partially disposed within handle 120.

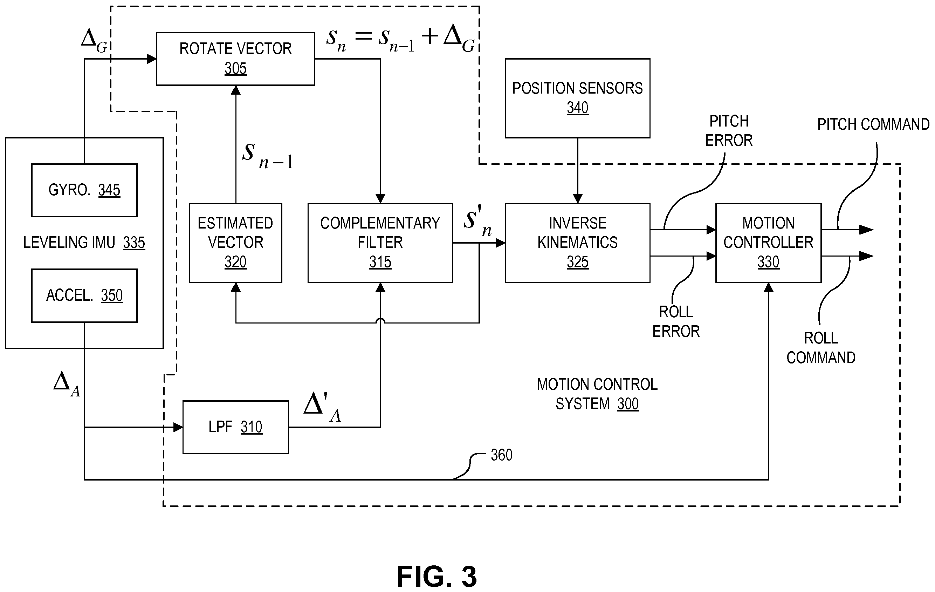

FIG. 3 is a functional block diagram illustrating functional components of a motion control system 300 for providing auto-leveling to user-assistive device 105 of a handheld tool 100, in accordance with an embodiment of the disclosure. Motion control system 300 is one possible implementation of motion control systems 150 or 205. Motion control system 300 may be implemented as software logic/instructions, as firmware logic/instructions, as hardware logic, or a combination thereof. In one embodiment, motion control system 300 is a DSP circuit.

The illustrated embodiment of motion control system 300 includes a rotate vector module 305, a low pass filter ("LPF") 310, a complementary filter module 315, an estimated vector module 320, an inverse kinematics module 325, and a motion controller 330. Motion control system 300 is coupled to receive feedback data from leveling IMU 335 and position sensors 340. The illustrated embodiment of leveling IMU 335 includes a gyroscope 345 and an accelerometer 350.

During operation, gyroscope 345 outputs gyro data Δ_{G} while accelerometer 350 outputs accelerometer data Δ_{A}. The gyro data Δ_{G} is used by rotate vector module 305 to adjust a previous error vector Sₙ₋₁ to generate a current error vector Sₙ. The current error vector Sₙ is then provided to complementary filter module 315. Complementary filter module 315 adjusts the current error vector Sₙ with a low pass filtered version Δ'_{A} of the accelerometer data Δ_{A} to generate an adjusted error vector S'ₙ. The adjust error vector S'ₙ is looped back to estimated vector module 320 where it is latched or temporarily stored and provided to rotated vector module 305 as the previous error vector Sₙ₋₁ for the next cycle of operation.

The adjusted error vector S'ₙ represents a difference vector between the frame of reference (e.g, gravity vector) and a vector representing the current position of user-assistive device 105. For example, the vector representing the current position of user-assistive device 105 may be a normal vector extending from a surface upon which leveling IMU 145 is disposed. Of course, other vector orientations for describing user-assistive device 105 may be used.

Gyroscope 345 is a rapid operation sensor that outputs angular velocity data quickly, but suffers from drift overtime. In contrast, accelerometer 350 is a slow sensor that outputs accurate readings that are used by complementary filter 315 to update the current error vector Sₙ and cancel out any drift. Accelerometer data Δ_{A} is low pass filtered to remove high frequency changes due to sudden jerks, such as tremor motions, which are less useful for the low frequency auto-leveling function.

The adjusted error vector S'ₙ is then provided to the inverse kinematics module 325. Inverse kinematics module 325 takes the adjusted error vector S'ₙ along with the current position information of actuator assembly 115 and generates error signals (e.g., pitch error and roll error) that define the position parameters of actuators 125 and 130 to obtain the desired position of user-assistive device 105. The use of kinematic equations are known in the field of robotic control systems.

The error signals are then input into motion controller 330, which determines how to implement the actual commands (e.g., pitch command and roll command) for controlling actuator assembly 115. In one embodiment, motion controller 330 is implemented as a proportional-integral-derivative ("PID") controller. Motion controller 330 attempts to reducing the error signals (e.g., pitch error and roll error) while also reducing correction overshoot and oscillations.

In the illustrated embodiment, motion control system 300 also includes a high frequency path 360 for accelerometer data Δ_{A} to reach motion controller 330. High frequency path 360 permits unfiltered high frequency accelerometer data Δ_{A} to be analyzed by motion controller 330 to implement tremor stability control.

Some of the functional logic/software explained above is described in terms of computer software and hardware. The techniques described may constitute machine-executable instructions embodied within a tangible or non-transitory machine (e.g., computer) readable storage medium, that when executed by a machine will cause the machine to perform the operations described. Additionally, the processes may be embodied within hardware, such as an application specific integrated circuit ("ASIC") or otherwise.

A tangible machine-readable storage medium includes any mechanism that provides (i.e., stores) information in a non-transitory form accessible by a machine (e.g., a computer, network device, personal digital assistant, manufacturing tool, any device with a set of one or more processors, etc.). For example, a machine-readable storage medium includes recordable/non-recordable media (e.g., read only memory (ROM), random access memory (RAM), magnetic disk storage media, optical storage media, flash memory devices, etc.).

The above description of illustrated embodiments of the invention, including what is described in the Abstract, is not intended to be exhaustive or to limit the invention to the precise forms disclosed. While specific embodiments of, and examples for, the invention are described herein for illustrative purposes, various modifications are possible within the scope of the invention, as those skilled in the relevant art will recognize.

These modifications can be made to the invention in light of the above detailed description. The terms used in the following claims should not be construed to limit the invention to the specific embodiments disclosed in the specification. Rather, the scope of the invention is to be determined entirely by the following claims, which are to be construed in accordance with established doctrines of claim interpretation.

## Claims

1. A handheld tool (100), comprising:
a handle (120) for holding by a user of the handheld tool;
an attachment arm (110) extending from the handle, the attachment arm configured to connect to a user-assistive device (105, 400);
a first inertial measurement unit ("IMU") (145, 175, 230, 240, 335, 340) mounted to the attachment arm to acquire measurements of one or more of a motion or an orientation of the user-assistive device and to generate feedback data indicative of the measurements;
an actuator assembly (115) coupled to manipulate the user-assistive device via the attachment arm in at least two dimensions;
a motion control system (150, 205, 300) coupled to receive the feedback data from the first IMU and coupled to provide commands to the actuator assembly to provide auto-leveling of the user-assistive device to a frame of reference while the user manipulates the handheld tool; and
**characterized in that** the actuator assembly includes:
a first actuator (125) and a second actuator (130),
wherein the first actuator is disposed within the handle and the second actuator is disposed external to the handle.

2. The handheld tool of claim 1, wherein the actuator assembly is coupled to manipulate the user-assistive device about two rotational axes relative to the handle.

3. The handheld tool of claim 2, wherein
the first actuator controls rolling motion of the attachment arm; and
the second actuator controls pitching motion of the attachment arm.

4. The handheld tool of claim 3, wherein the actuator assembly further comprises:
a first linkage (135) that mechanically couples the rolling motion output from the first actuator (125) to the second actuator (130), wherein the first actuator is coupled to roll the attachment arm by rolling the second actuator via the first linkage; and
a second linkage (140) that mechanically couples the pitching motion output from the second actuator (130) to the attachment arm.

5. The handheld tool of claim 3, further comprising:
a first position sensor coupled to monitor a first relative position of a first output of the first actuator; and
a second position sensor coupled to monitor a second relative position of a second output of the second actuator,
wherein the first and second position sensors are coupled to feedback position information to the motion control system.

6. The handheld tool of claim 5, further comprising:
a system controller (160, 215) disposed within the handle and coupled to collect position information from the first and second position sensors and to record the position information into a log; and
communication interface coupled to the system controller to output the log from the handheld tool.

7. The handheld tool of claim 2, wherein the motion control system is further coupled to the actuator assembly to manipulate the actuator assembly about the two rotational axes to provide human tremor stabilization to the user-assistive device.

8. The handheld tool of claim 1, further comprising a power supply coupled to power the actuator assembly and the motion control system, wherein the power supply, the motion control system, and at least a portion of the actuator assembly are disposed within the handle.

9. The handheld tool of claim 1, wherein the user-assistive device comprises either one of an eating utensil or a drinking utensil.

10. The handheld tool of claim 1, wherein the user-assistive device comprises one of a pointer device, a grooming tool, a personal hygiene tool, or an occupational tool.

11. The handheld tool of claim 1, further comprising:
a second IMU rigidly connected to the handle to measure human tremors while the user holds and uses the handheld tool.

12. A method executed by a handheld tool (100), the method comprising:
measuring at least one of a motion or an orientation of a user-assistive device (105, 400) mounted to a distal end of the handheld tool with an inertial measurement unit ("IMU") (145, 175, 230, 240, 335, 340);
outputing feedback data from the IMU based upon the measuring;
monitoring the feedback data in real-time with a motion control system (150, 205, 300) at least partially disposed within a handle (120) of the handheld tool;
controlling an actuator assembly (115) with the motion control system, wherein the actuator assembly is coupled to manipulate the user-assistive device in at least two orthogonal dimensions to provide auto-leveling of the user-assistive device to a frame of reference while a user manipulates the handheld tool; and
**characterised in that** the actuator assembly includes:
a first actuator (125) and a second actuator (130),
wherein the first actuator is disposed within the handle and the second actuator is disposed external to the handle.

13. The method of claim 12, wherein the at least two orthogonal dimensions comprise two rotational axes including a pitch axis and a roll axis.

14. The method of claim 13, wherein controlling the actuator assembly with the motion control system to provide auto-leveling comprises:
generating an error vector indicating a positional deviation of the user-assistive device from a reference vector based upon the frame of reference;
updating the error vector based upon the feedback data output from the IMU; and
generating a pitch command to manipulate the actuator assembly about the pitch axis and a roll command to manipulate the actuator assembly about the roll axis based at least in part upon the error vector.

15. The method of claim 14, wherein the IMU includes a gyroscope and an accelerometer, wherein the feedback data includes gyroscope feedback data and accelerometer feedback data, and wherein controlling the actuator assembly with the motion control system to provide auto-leveling further comprises:
updating the error vector with the gyroscope feedback data; and
low pass filtering the accelerometer feedback data before updating the error vector with the accelerometer feedback data.

16. The handheld tool of claim 4, wherein the second linkage is external to the handle.

17. The handheld tool of claim 1, wherein the first IMU is rigidly mounted to the attachment arm.

## Patentansprüche

1. Handwerkzeug (100), umfassend:
einen Griff (120) zum Halten des Handwerkzeugs durch einen Benutzer;
einen Befestigungsarm (110), der sich vom Griff aus erstreckt, wobei der Befestigungsarm ausgelegt ist, um mit einer Benutzerhilfsvorrichtung (105, 400) eine Verbindung herzustellen;
eine erste inertiale Messeinheit ("IMU") (145, 175, 230, 240, 335, 340), die am Befestigungsarm angebracht ist, um Messungen einer oder mehrerer aus einer Bewegung oder einer Ausrichtung der Benutzerhilfsvorrichtung zu erhalten und Rückmeldungsdaten zu erzeugen, die die Messungen angeben;
eine Aktoranordnung (115), die gekoppelt ist zum Betätigen der Benutzerhilfsvorrichtung über den Befestigungsarm in zumindest zwei Dimensionen;
ein Bewegungssteuerungssystem (150, 205, 300), das gekoppelt ist zum Empfangen der Rückmeldungsdaten von der ersten IMU und gekoppelt ist zum Bereitstellen von Befehlen an die Aktoranordnung, um automatische Höhenregulierung der Benutzerhilfsvorrichtung gegenüber einem Bezugssystem bereitzustellen, während der Benutzer das Handwerkzeug betätigt; und
**dadurch gekennzeichnet, dass** die Aktoranordnung Folgendes umfasst:
einen ersten Aktor (125) und einen zweiten Aktor (130),
wobei der erste Aktor innerhalb des Griffs angeordnet ist und der zweite Aktor außerhalb des Griffs angeordnet ist.

2. Handwerkzeug nach Anspruch 1, wobei die Aktoranordnung gekoppelt ist zum Betätigen der Benutzerhilfsvorrichtung um zwei Drehachsen relativ zum Griff.

3. Handwerkzeug nach Anspruch 2, wobei
der erste Aktor eine Rollbewegung des Befestigungsarms steuert; und
der zweite Aktor eine Nickbewegung des Befestigungsarms steuert.

4. Handwerkzeug nach Anspruch 3, wobei die Aktoranordnung ferner Folgendes umfasst:
eine erste Kopplung (135), die die vom ersten Aktor (125) ausgegebene Rollbewegung mechanisch mit dem zweiten Aktor (130) koppelt, wobei der erste Aktor gekoppelt ist zum Rollen des Befestigungsarms durch Rollen des zweiten Aktors über die erste Kopplung; und
eine zweite Kopplung (140), die die vom zweiten Aktor (130) ausgegebene Nickbewegung mechanisch mit dem Befestigungsarm koppelt.

5. Handwerkzeug nach Anspruch 3, ferner umfassend:
einen ersten Positionssensor, der gekoppelt ist zum Überwachen einer ersten relativen Position eines ersten Ausgangs des ersten Aktors; und
einen zweiten Positionssensor, der gekoppelt ist zum Überwachen einer zweiten relativen Position eines zweiten Ausgangs des zweiten Aktors,
wobei der erste und der zweite Positionssensor gekoppelt sind zum Rückmelden von Positionsinformationen an das Bewegungssteuerungssystem.

6. Handwerkzeug nach Anspruch 5, ferner umfassend:
eine Systemsteuerung (160, 215), die innerhalb des Griffs angeordnet ist und gekoppelt ist zum Erfassen von Positionsinformationen von dem ersten und zweiten Positionssensor und zum Aufzeichnen der Positionsinformationen in ein Protokoll; und
eine Kommunikationsschnittstelle, die mit der Systemsteuerung gekoppelt ist, um das Protokoll aus dem Handwerkzeug auszugeben.

7. Handwerkzeug nach Anspruch 2, wobei das Bewegungssteuerungssystem ferner mit der Aktoranordnung gekoppelt ist, um die Aktoranordnung um die zwei Drehachsen zu betätigen, um der Benutzerhilfsvorrichtung eine Stabilisierung von menschlichem Tremor bereitzustellen.

8. Handwerkzeug nach Anspruch 1, ferner umfassend eine Leistungsversorgung, die gekoppelt ist zum Versorgen der Aktoranordnung und des Bewegungssteuerungssystems, wobei die Leistungsversorgung, das Bewegungssteuerungssystem und zumindest ein Abschnitt der Aktoranordnung innerhalb des Griffs angeordnet sind.

9. Handwerkzeug nach Anspruch 1, wobei die Benutzerhilfsvorrichtung entweder ein Essbesteck oder ein Trinkbesteck umfasst.

10. Handwerkzeug nach Anspruch 1, wobei die Benutzerhilfsvorrichtung eines aus einer Zeigevorrichtung, einem Körperpflegewerkzeug, einem Körperhygienewerkzeug oder einem Berufswerkzeug umfasst.

11. Handwerkzeug nach Anspruch 1, ferner umfassend:
eine zweite IMU, die mit dem Griff fest verbunden ist, um menschliche Tremores zu messen, während der Benutzer das Handwerkzeug hält und benutzt.

12. Verfahren, das von einem Handwerkzeug (100) ausgeführt wird, wobei das Verfahren Folgendes umfasst:
Messen zumindest eines aus einer Bewegung oder einer Ausrichtung einer Benutzerhilfsvorrichtung (105, 400), die an einem distalen Ende des Handwerkzeugs angebracht ist, mit einer inertialen Messeinheit ("IMU") (145, 175, 230, 240, 335, 340);
Ausgeben von Rückmeldungsdaten aus der IMU auf Basis der Messung;
Überwachen der Rückmeldungsdaten in Echtzeit mit einem Bewegungssteuerungssystem (150, 205, 300), das zumindest teilweise innerhalb eines Griffs (120) des Handwerkzeugs angeordnet ist;
Steuern einer Aktoranordnung (115) mit dem Bewegungssteuerungssystem, wobei die Aktoranordnung gekoppelt ist zum Betätigen der Benutzerhilfsvorrichtung in zumindest zwei orthogonalen Dimensionen, um automatische Höhenregulierung der Benutzerhilfsvorrichtung gegenüber einem Bezugssystem bereitzustellen, während ein Benutzer das Handwerkzeug betätigt; und
**dadurch gekennzeichnet, dass** die Aktoranordnung Folgendes beinhaltet:
einen ersten Aktor (125) und einen zweiten Aktor (130),
wobei der erste Aktor innerhalb des Griffs angeordnet ist und der zweite Aktor außerhalb des Griffs angeordnet ist.

13. Verfahren nach Anspruch 12, wobei die zumindest zwei orthogonalen Dimensionen zwei Drehachsen, einschließlich einer Nickachse und einer Rollachse, umfassen.

14. Verfahren nach Anspruch 13, wobei das Steuern der Aktoranordnung mit dem Bewegungssteuerungssystem zum Bereitstellen von automatischer Höhenregulierung Folgendes umfasst:
Erzeugen eines Fehlervektors, der eine Positionsabweichung der Benutzerhilfsvorrichtung von einem Bezugsvektor auf Basis des Bezugssystems angibt;
Aktualisieren des Fehlervektors auf Basis der aus der IMU ausgegebenen Rückmeldungsdaten; und
Erzeugen eines Nickbefehls zum Betätigen der Aktoranordnung um die Nickachse und eines Rollbefehls zum Betätigen der Aktoranordnung um die Rollachse zumindest teilweise auf Basis des Fehlervektors.

15. Verfahren nach Anspruch 14, wobei die IMU ein Gyroskop und einen Beschleunigungsmesser beinhaltet, wobei die Rückmeldungsdaten Gyroskop-Rückmeldungsdaten und Beschleunigungsmesser-Rückmeldungsdaten beinhalten und wobei das Steuern der Aktoranordnung mit dem Bewegungssteuerungssystem zum Bereitstellen von automatischer Höhenregulierung ferner Folgendes umfasst:
Aktualisieren des Fehlervektors mit den Gyroskop-Rückmeldungsdaten; und
Tiefpassfiltern der Beschleunigungsmesser-Rückmeldungsdaten, bevor der Fehlervektor mit den Beschleunigungsmesser-Rückmeldungsdaten aktualisiert wird.

16. Handwerkzeug nach Anspruch 4, wobei die zweite Kopplung außerhalb des Griffs angeordnet ist.

17. Handwerkzeug nach Anspruch 1, wobei die erste IMU fest an dem Befestigungsarm angebracht ist.

## Revendications

1. Outil à main (100), comprenant :
une poignée (120) destinée à être tenue par un utilisateur de l'outil à main ;
un bras de fixation (110) qui s'étend depuis la poignée, le bras de fixation étant configuré pour être relié à un dispositif d'assistance utilisateur (105, 400) ;
une première unité de mesure inertielle (« IMU ») (145, 175, 230, 240, 335, 340) montée sur le bras de fixation afin d'acquérir des mesures d'un ou plusieurs d'un mouvement ou d'une orientation du dispositif d'assistance utilisateur et de générer des données de retour qui indiquent les mesures ;
un ensemble d'actionneur (115) relié afin de manipuler le dispositif d'assistance utilisateur via le bras de fixation dans au moins deux dimensions ;
un système de commande de mouvement (150, 205, 300) relié afin de recevoir les données de retour de la part de la première IMU et relié afin de fournir des commandes à l'ensemble d'actionneur de façon à assurer un nivellement automatique du dispositif d'assistance utilisateur par rapport à un cadre de référence pendant que l'utilisateur manipule l'outil à main ; et
**caractérisé en ce que** l'ensemble d'actionneur comprend :
un premier actionneur (125) et un second actionneur (130),
dans lequel le premier actionneur est disposé dans la poignée et le second actionneur est disposé de manière externe à la poignée.

2. Outil à main selon la revendication 1, dans lequel l'ensemble d'actionneur est relié afin de manipuler le dispositif d'assistance utilisateur autour de deux axes de rotation par rapport à la poignée.

3. Outil à main selon la revendication 2, dans lequel
le premier actionneur contrôle le mouvement de roulis du bras de fixation ; et
le second actionneur contrôle le mouvement de tangage du bras de fixation.

4. Outil à main selon la revendication 3, dans lequel l'ensemble d'actionneur comprend en outre :
une première liaison (135) qui couple mécaniquement le mouvement de roulis transmis par le premier actionneur (125) au second actionneur (130), dans lequel le premier actionneur est couplé afin de faire rouler le bras de fixation en faisant rouler le second actionneur via la première liaison ; et
une seconde liaison (140) qui couple mécaniquement le mouvement de tangage transmis par le second actionneur (130) au bras de fixation.

5. Outil à main selon la revendication 3, comprenant en outre :
un premier capteur de position couplé pour surveiller une première position relative d'une première sortie du premier actionneur ; et
un second capteur de position couplé pour surveiller une seconde position relative d'une seconde sortie du second actionneur,
dans lequel le premier et le second capteurs de position sont couplés afin de fournir des informations de position au système de contrôle de mouvement.

6. Outil à main selon la revendication 5, comprenant en outre :
un contrôleur de système (160, 215) disposé dans la poignée et couplé afin de collecter les informations de position issues du premier et du second capteurs de position et d'enregistrer les informations de position dans un journal ; et
une interface de communication couplée au contrôleur de système afin de fournir le journal issu de l'outil à main.

7. Outil à main selon la revendication 2, dans lequel le système de contrôle de mouvement est en outre couplé à l'ensemble d'actionneur afin de manipuler l'ensemble d'actionneur autour des deux axes de rotation de façon à offrir une stabilisation de tremblement humain au dispositif d'assistance utilisateur.

8. Outil à main selon la revendication 1, comprenant en outre une alimentation électrique couplée afin d'alimenter l'ensemble d'actionneur et le système de contrôle de mouvement, dans lequel l'alimentation électrique, le système de contrôle de mouvement et au moins une partie de l'ensemble d'actionneur sont disposés dans la poignée.

9. Outil à main selon la revendication 1, dans lequel le dispositif d'assistance utilisateur comprend l'un d'un ustensile d'alimentation ou de boisson.

10. Outil à main selon la revendication 1, dans lequel le dispositif d'assistance utilisateur comprend l'un d'un dispositif de pointage, d'un outil de toilette, d'un outil d'hygiène personnelle, ou d'un outil professionnel.

11. Outil à main selon la revendication 1, comprenant en outre :
une seconde IMU reliée de manière rigide à la poignée afin de mesurer les tremblements humains pendant que l'utilisateur tient et utilise l'outil à main.

12. Procédé exécuté par un outil à main (100), le procédé comprenant :
la mesure d'au moins l'un d'un mouvement ou d'une orientation d'un dispositif d'assistance utilisateur (105, 400) monté sur une extrémité distale de l'outil à main avec une unité de mesure inertielle (« IMU ») (145, 175, 230, 240, 335, 340) ;
la délivrance des données de retour de l'IMU sur la base de la mesure ;
la surveillance des données de retour en temps réel avec un système de contrôle de mouvement (150, 205, 300) au moins partiellement disposé dans une poignée (120) de l'outil à main ;
le contrôle d'un ensemble d'actionneur (115) avec le système de contrôle de mouvement, dans lequel l'ensemble d'actionneur est couplé afin de manipuler le dispositif d'assistance utilisateur dans au moins deux dimensions orthogonales de façon à assurer le nivellement automatique du dispositif d'assistance utilisateur par rapport à un cadre de référence pendant qu'un utilisateur manipule l'outil à main ; et
**caractérisé en ce que** l'ensemble d'actionneur comprend :
un premier actionneur (125) et un second actionneur (130),
dans lequel le premier actionneur est disposé dans la poignée et le second actionneur est disposé de manière externe à la poignée.

13. Procédé selon la revendication 12, dans lequel au moins deux dimensions orthogonales comprennent deux axes de rotation comprenant un axe de tangage et un axe de roulis.

14. Procédé selon la revendication 13, dans lequel le contrôle de l'ensemble d'actionneur avec le système de contrôle de mouvement destiné à assurer un nivellement automatique comprend :
la génération d'un vecteur d'erreur qui indique un écart de position du dispositif d'assistance utilisateur à partir d'un vecteur de référence sur la base du cadre de référence ;
la mise à jour du vecteur d'erreur sur la base des données de retour délivrées par l'IMU ; et
la génération d'une commande de tangage afin de manipuler l'ensemble d'actionneur autour de l'axe de tangage et d'une commande de roulis afin de manipuler l'ensemble d'actionneur autour de l'axe de roulis sur la base au moins en partie du vecteur d'erreur.

15. Procédé selon la revendication 14, dans lequel l'IMU comprend un gyroscope et un accéléromètre, dans lequel les données de retour comprennent des données de retour de gyroscope et des données de retour d'accéléromètre, et dans lequel le contrôle de l'ensemble d'actionneur avec le système de contrôle de mouvement afin d'assurer un nivellement automatique comprend en outre :
la mise à jour du vecteur d'erreur avec les données de retour de gyroscope ; et
le filtrage passe-bas des données de retour d'accéléromètre afin de mettre à jour le vecteur d'erreur avec les données de retour d'accéléromètre.

16. Outil à main selon la revendication 4, dans lequel la seconde liaison est externe à la poignée.

17. Outil à main selon la revendication 1, dans lequel l'IMU est montée de manière rigide sur le bras de fixation.
